# EUROPEAN PATENT APPLICATION

(11) **EP 4 505 991 A1**
(43) Date of publication of application: **12.02.2025**
(21) Application number: 23780687.2
(22) Date of filing: 29.03.2023
(51) Int. Cl.: A61K 6/30

(54) **ADHESIVE COMPOSITION FOR DENTAL USE**

(30) Priority: 31.03.2022 JP 2022060618
(71) Applicant: GC Corporation, Sunto-gun, Shizuoka 410-1307 (JP)
(72) Inventor: HIGASHI, Sayaka, Tokyo 174-8585 (JP); SATO, Yuna, Tokyo 174-8585 (JP); SHOJI, Takumi, Tokyo 174-8585 (JP)
(74) Representative: Müller-Boré & Partner Patentanwälte PartG mbB
(86) International application number: PCT/JP2023/012785
(87) International publication number: WO 2023/190678

(57) **Abstract**

A dental adhesive composition provided as a two-part formulation including a first agent and a second agent, wherein the first agent contains a (meth) acrylate having an acid group and a linear siloxane represented by a general formula (1) 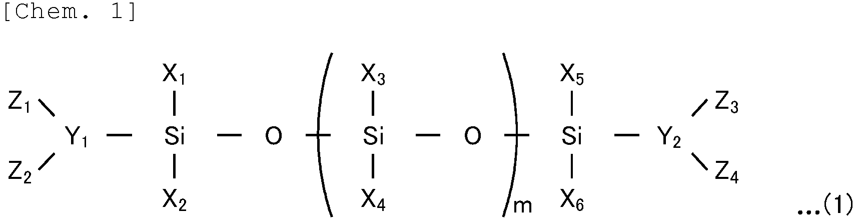 (in the general formula (1), X₁ to X₆ are independently an alkyl group or an ethylenically unsaturated group having 1 or more and 6 or less carbon atoms; m is an integer of 0 or more and 500 or less; in a case where m is 2 or more, X₃ and X₄ are same or different for each repeating unit; Y₁ and Y₂ are independently a linear hydrocarbon group or a branched hydrocarbon group having 1 or more and 20 or less carbon atoms; Z₁ to Z₄ are independently a hydrogen atom or an ethylenically unsaturated group; and at least one of X₁ to X₆ or Z₁ to Z₄ is an ethylenically unsaturated group), and the second agent contains a (meth)acrylate.

## Description

### Technical Field

The present invention relates to a dental adhesive composition.

### Background Art

A dental adhesive composition provided as a two-part formulation is known to exhibit better adhesive strength to both tooth and composite resin than a dental adhesive composition provided as a single-part formulation.

As a dental adhesive composition provided as a two-part formulation, a dental adhesive system is disclosed that includes a primer composition consisting of (a) a polymerizable monomer having an acidic group, (b) a polymerizable monomer having a hydroxyl group, and (c) water, and a bonding composition consisting of (d) an acylphosphine oxide compound, and (e) a polymerizable monomer, for example (see Patent Document 1).

### Citation List

### Patent Document

Patent Document 1: Japanese Unexamined Patent Application Publication No. 2000-016911

### Summary of Invention

### Technical Problem

However, in the conventional dental adhesive composition, adhesive strength to ceramics is not sufficient.

In view of the problems associated with the above conventional technologies, an object of an aspect of the present invention is to provide a dental adhesive composition having excellent adhesive strength to ceramics.

### Solution to Problem

According to one aspect of the present invention, a dental adhesive composition provided as a two-part formulation including a first agent and a second agent is provided. The first agent contains a (meth)acrylate having an acid group and a linear siloxane represented by a general formula (1), and the second agent contains a (meth)acrylate.

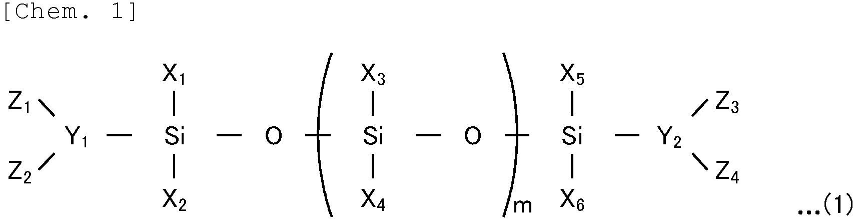

(In the general formula (1), X₁ to X₆ are independently an alkyl group or an ethylenically unsaturated group having 1 or more and 6 or less carbon atoms; m is an integer of 0 or more and 500 or less; in a case where m is 2 or more, X₃ and X₄ are same or different for each repeating unit; Y₁ and Y₂ are independently a linear hydrocarbon group or a branched hydrocarbon group having 1 or more and 20 or less carbon atoms; Z₁ to Z₄ are independently a hydrogen atom or an ethylenically unsaturated group; and at least one of X₁ to X₆ or Z₁ to Z₄ is an ethylenically unsaturated group.)

### Advantageous Effects of Invention

According to one aspect of the present disclosure, a dental adhesive composition having excellent adhesive strength to ceramics can be provided.

### Description of Embodiments

In the following, embodiments of the present invention will be described in detail.

### [Dental Adhesive Composition]

The dental adhesive composition according to the present embodiment is a dental adhesive composition provided as a two-part formulation including a first agent and a second agent. The dental adhesive composition according to the present embodiment may be composed of only the first agent and the second agent.

Hereinafter, the composition of the first agent and the second agent will be described.

### (1) First Agent

In the dental adhesive composition provided as a two-part formulation, the first agent is also referred to as a pretreatment agent or a primer. The first agent contains a (meth)acrylate having an acid group and a linear siloxane having a predetermined structure.

As used herein, a (meth)acrylate refers to a monomer, oligomer, or prepolymer having one or more (meth)acryloyloxy groups of (meth)acrylates. A (meth)acrylate refers to one or both of an acrylate and a methacrylate.

The siloxane is a structure represented by -silicon (Si)-oxygen (O)-[-silicon (Si)-oxygen (O)-]_{L}-silicon (Si)- (L is an integer of 0 or more).

Hereinafter, each component contained in the first agent will be described.

### (1-1) Component Contained in First Agent

### (A) (Meth)acrylate Having Acid Group

The (meth)acrylate having an acid group contained in the first agent is not particularly limited, but for example, a (meth)acrylate having an acid group of a phosphate group, a pyrophosphate group, a thiophosphate group, a carboxylic acid group, a sulfonic acid group, a phosphonic acid group, and the like may be preferably used. The (meth)acrylate having an acid group may include a plurality of acid groups.

Examples of the (meth)acrylate having a phosphate group include 2-(meth)acryloyloxyethyl dihydrogenphosphate, bis[2-(meth)acryloyloxyethyl] hydrogenphosphate, 2-(meth)acryloyloxyethyl phenyl hydrogenphosphate, 6-(meth)acryloyloxyhexyl dihydrogenphosphate, 6-(meth)acryloyloxyhexyl phenyl hydrogenphosphate, 10-(meth)acryloyloxydecyl dihydrogenphosphate, 1,3-di(meth)acryloylopropane-2 dihydrogenphosphate, 1,3-di(meth)acryloylopropane-2 phenyl hydrogenphosphate, bis[5-{2-(meth)acryloyloxyethoxycarbonyl} heptyl] hydrogenphosphate, and the like.

Examples of the (meth)acrylate having a pyrophosphate group include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, and the like.

Examples of the (meth)acrylate having a thiophosphate group include 2-(meth)acryloyloxyethyl dihydrogenthiophosphate, 3-(meth)acryloyloxypropyl dihydrogenthiophosphate, 4-(meth)acryloyloxybutyl dihydrogenthiophosphate, 5-(meth)acryloyloxypentyl dihydrogenthiophosphate, 6-(meth)acryloyloxyhexyl dihydrogenthiophosphate, 7-(meth)acryloyloxyheptyl dihydrogenthiophosphate, 8-(meth)acryloyloxyoctyl dihydrogenthiophosphate, 9-(meth)acryloyloxynonyl dihydrogenthiophosphate, 10 (meth)acryloyloxydecyl dihydrogenthiophosphate, 11-(meth)acryloyloxyundecyl dihydrogenthiophosphate, 12-(meth)acryloyloxydodecyl dihydrogenthiophosphate, 13-(meth)acryloyloxytridecyl dihydrogenthiophosphate, 14-(meth)acryloyloxytetradecyl dihydrogenthiophosphate, 15-(meth)acryloyloxypentadecyl dihydrogenthiophosphate, 16-(meth)acryloyloxyhexadecyl dihydrogenthiophosphate, 17-(meth)acryloyloxyheptadecyl dihydrogenthiophosphate, 18-(meth)acryloyloxyoctadecyl dihydrogenthiophosphate, 19-(meth)acryloyloxynonadecyl dihydrogenthiophosphate, 20-(meth)acryloyloxyicosyl dihydrogenthiophosphate, and the like.

Examples of the (meth)acrylate having a carboxylic acid group include 2-methacryloyloxyethyl succinic acid, 4-(meth)acryloyloxyethyl trimellitic acid, 4-(meth)acryloyloxyethyl trimellitic anhydride, 4-(meth)acryloyloxydecyl trimellitic acid, 4-(meth)acryloyloxydecyl trimellitic anhydride, 11-(meth)acryloyloxy-1,1-undecandicarboxylic acid, 1,4-di(meth)acryloyloxypyromellitic acid, 2-(meth)acryloyloxyethyl maleic acid, 2-(meth)acryloyloxyethyl phthalic acid, 2-(meth)acryloyloxyethyl hexahydrophthalic acid, and the like.

Examples of the (meth)acrylate having a sulfonic acid group include 2-(meth)acrylamide-2 methylpropanesulfonic acid, styrene sulfonic acid, 2-sulfoethyl (meth)acrylate, and the like.

Examples of the (meth)acrylate having a phosphonic acid group include 2-(meth)acryloyloxyethyl phenylphosphonate, 5-(meth)acryloyloxypentyl-3 phosphonopropionate, 6-(meth)acryloyloxyhexyl-3 phosphonopropionate, 10-(meth)acryloyloxydecyl-3 phosphonopropionate, 6-(meth)acryloyloxyhexyl-3 phosphonoacetate, 10-(meth)acryloyloxydecyl-3 phosphonoacetate, and the like.

The (meth)acrylate having an acid group may be used alone or in combination of two or more.

Among these, as the (meth)acrylate having an acid group, a (meth)acrylate having one or more groups selected from a phosphate group, a thiophosphate group, and a carboxylic acid group is preferable, in terms of solubility of the smear layer on the tooth surface and tooth demineralization of the dental adhesive composition, especially in terms of adhesiveness to enamel.

Further, among these, as the (meth)acrylate having an acid group, one or more (meth)acrylates selected from 10-methacryloyloxydecyl dihydrogenphosphate (MDP), 10-methacryloyloxydecyl dihydrogenthiophosphate (MDTP), 4-methacryloyloxyethyl trimellitic anhydride (4-META), and the like are preferable, in terms of improving adhesiveness of the dental adhesive composition.

The content of the (meth)acrylate having an acid group in the first agent is not particularly limited, but is, for example, preferably 0.1 mass% or more and 40 mass% or less, more preferably 1 mass% or more and 25 mass% or less, and still more preferably 5 mass% or more and 25 mass% or less, based on 100 mass% of the first agent.

When the first agent contains a plurality of types of (meth)acrylates having an acid group, it is preferable that the total content satisfies the above range.

When the content of the (meth)acrylate having an acid group in the first agent is 0.1 mass% or more, the demineralization power of the dental adhesive composition to the tooth is further improved, and when the content is 40 mass% or less, the curability of the dental adhesive composition is improved.

### (B) Linear Siloxane

The linear siloxane contained in the first agent is represented by the following general formula (1).

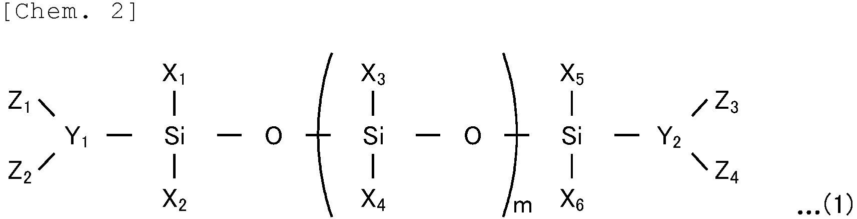

In the general formula (1), X₁ to X₆ are independently an alkyl group or an ethylenically unsaturated group having 1 or more and 6 or less carbon atoms. Here, the independently means that the structures and carbon atoms of X₁ to X₆ may be different from each other.

The upper limit of the carbon atoms in X₁ to X₆ is preferably 4, more preferably 3, and still more preferably 2.

Examples of the alkyl group having 1 or more and 6 or less carbon atoms of X₁ to X₆ include, but are not particularly limited to, a methyl group, an ethyl group, a propyl group, an isopropyl group, a butyl group, a sec-butyl group, an isobutyl group, a tert-butyl group, a pentyl group, an isopentyl group, a sec-pentyl group, a tert-pentyl group, a neopentyl group, a hexyl group, an isohexyl group, a 3-methylpentyl group, a 2-methylpentyl group, a 2-ethylbutyl group, a 1,2-dimethylbutyl group, a 2,3-dimethylbutyl group, an n-heptyl group, and the like.

Examples of the ethylenically unsaturated group include, but are not particularly limited to, a vinyl group, an allyl group, an acrylic group (also referred to as an acryloyl group), a methacrylic group (also referred to as a methacryloyl group), and the like. The ethylenically unsaturated group may include an alkylene group having 1 or more and 6 or less carbon atoms.

In the general formula (1), m is an integer of 0 or more and 500 or less, preferably 0 or more and 300 or less, more preferably 0 or more and 100 or less, still more preferably 0 or more and 50 or less, and particularly preferably 0 or more and 10 or less.

In a case where m is 2 or more, X₃ and X₄ may be the same or different for each repeating unit. Specifically, for example, when m is 2, one of the repeating units may be of alkyl groups having 1 carbon atom in X₃ and X₄, and the other one of the repeating units may be of alkyl groups having 2 carbon atoms in X₃ and X₄.

In the general formula (1), Y₁ and Y₂ are independently a linear hydrocarbon group or a branched hydrocarbon group having 1 or more and 20 or less carbon atoms. Y₁ and Y₂ may include an ether bond.

The term "a linear hydrocarbon group or a branched hydrocarbon group having 1 or more and 20 or less carbon atoms" refers to a linear hydrocarbon group having 1 or more and 20 or less carbon atoms or a branched hydrocarbon group having 1 or more and 20 or less carbon atoms.

The upper limit of the carbon atoms in Y₁ and Y₂ is preferably 16, more preferably 8, and still more preferably 2.

Examples of the linear hydrocarbon group or the branched hydrocarbon group having 1 or more and 20 or less carbon atoms of Y₁ and Y₂ include, but are not particularly limited to, structures represented by C_{α}H_{2α} and C_{α}H_{2α-1} (α is an integer of 1 or more and 20 or less).

In the general formula (1), Z₁ to Z₄ are independently a hydrogen atom or an ethylenically unsaturated group. For example, when one of Z₁ and Z₂ is hydrogen and the other is the ethylenically unsaturated group, the ethylenically unsaturated group is an end group of the linear siloxane represented by the general formula (1). When both Z₁ and Z₂ are hydrogen atoms, Y₁ is the end group of the linear siloxane represented by the general formula (1).

When one of Z₃ and Z₄ is hydrogen and the other is the ethylenically unsaturated group, the ethylenically unsaturated group is the end group of the linear siloxane represented by the general formula (1). When both Z₃ and Z₄ are hydrogen atoms, Y₂ is the end group of the linear siloxane represented by the general formula (1).

In the general formula (1), at least one of X₁ to X₆ and Z₁ to Z₄ is the ethylenically unsaturated group.

That is, the linear siloxane represented by the general formula (1) may include the ethylenically unsaturated group at one end of the siloxane, may include the ethylenically unsaturated groups at both ends of the siloxane, and may include the ethylenically unsaturated group at the side chain.

It is preferable when the linear siloxane represented by the general formula (1) includes the ethylenically unsaturated group at the side chain, because a surface modification effect is obtained, crosslinking density is improved, and hardness is increased.

When the siloxane includes the ethylenically unsaturated group at one end or both ends of the siloxane, a surface modification effect is obtained, crosslinking density is improved, and hardness is increased.

Specific examples of the linear siloxane having at least one ethylenically unsaturated group include a component having (meth)acryloyl groups at both ends of a polysiloxane ("X-22-164", "X-22-164AS", "X-22-164A", "X-22-164B", "X-22-164C", "X-22-164E", "KP-410", "KP-411", "KP-412", "KP-413", "KP-414", "KP-415", and "KP-423", manufactured by Shin-Etsu Chemical Co., Ltd., and "DMS-U21" manufactured by Gelest, Inc.); a component having a (meth)acryloyloxy group at one end of a polysiloxane ("X-22-174ASX", "X-22-174BX", "KF-2012", "X-22-2426", "X-22-2404", "KP-416", "KP-418", and "KP-422", manufactured by Shin-Etsu Chemical Co., Ltd.); and a component having a (meth)acryloyloxy group at a side chain of a polysiloxane ("KP-420" manufactured by Shin-Etsu Chemical Co., Ltd.); and the like.

The linear siloxane may be used alone, or in combination of two or more.

The content of the linear siloxane in the first agent is not particularly limited, but is, for example, preferably 0.01 mass% or more and 30 mass% or less, more preferably 0.05 mass% or more and 25 mass% or less, and still more preferably 0.1 mass% or more and 20 mass% or less, based on 100 mass% of the first agent.

When the first agent contains a plurality of types of linear siloxanes, it is preferable that the total content satisfies the above range.

When the content of the linear siloxane in the first agent is 0.01 mass% or more, the adhesive strength to the ceramics is improved by lowering the surface tension, and when the content is 30 mass% or less, the hydrophilicity of the dental adhesive composition is maintained and the adhesiveness to the tooth is improved.

The first agent may contain other components as long as they do not detract from the purpose of the present invention. Examples of the other components contained in the first agent include a (meth)acrylate having no acid group, a polymerization initiator, a polymerization inhibitor, a filler, and a solvent. Examples of the polymerization initiator include a chemical polymerization initiator and a photopolymerization initiator.

Hereinafter, the above components will also be described.

### (C) (Meth)acrylate Having No Acid Group

The first agent may contain the (meth)acrylate having no acid group.

The (meth)acrylate having no acid group is not particularly limited. Examples of the (meth)acrylate having no acid group include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-methylhexyl (meth)acrylate, 2-ethylhexyl (meth)acrylate, benzyl (meth)acrylate, 2-hydroxy-1,3 di(meth)acryloyloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra (meth)acrylate, polybutylene glycol di(meth)acrylate, bisphenol A diglycidyl (meth)acrylate, di-2-(meth)acryloyloxyethyl-2,2,4-trimethylhexamethylenedicarbamate, 1,3,5-tris[1,3-bis{ (meth)acryloyloxy}-2-propoxycarbonylaminohexane]-1,3,5-(1H,3H,5H) triazine-2,4,6-trione, 2,2-bis[4-(3-(meth)acryloyloxy-2-hydroxypropyl) phenyl] propane, N,N'-(2,2,4-trimethylhexamethylene) bis[2-(aminocarboxy) propane-1,3-diol] tetramethacrylate, and the like.

The (meth)acrylate having no acid group may be used alone, or in combination of two or more.

Among these, as the (meth)acrylates having no acid group, 2-hydroxy-1,3-dimethacryloyloxypropane and triethylene glycol dimethacrylate are preferable, in terms of improving the mechanical strength of the cured body of the dental adhesive composition and improving the adhesiveness to the ceramics and to the tooth.

The content of the (meth)acrylate having no acid group in the first agent is preferably 3 mass% or more and 50 mass% or less, more preferably 5 mass% or more and 40 mass% or less, and still more preferably 10 mass% or more and 30 mass% or less, based on 100 mass% of the first agent.

When the first agent contains a plurality of types of (meth)acrylates having no acid group, it is preferable that the total content satisfies the above range.

When the content of the (meth)acrylate having no acid group in the first agent is 3 mass% or more, the operability of the dental adhesive composition can be improved. When the content of the (meth)acrylate having no acid group in the first agent is 50 mass% or less, the mechanical strength of the dental polymerizable composition can be improved. (D) Chemical Polymerization Initiator

The chemical polymerization initiator is not particularly limited, but for example, one or more compounds selected from a thiourea derivative, a vanadium compound, and an organic peroxide may be used.

### (D-1) Thiourea Derivative

Among the chemical polymerization initiators, the thiourea derivative functions as a reducing agent.

Examples of the thiourea derivative include, but are not particularly limited to, ethylenethiourea, N-methylthiourea, N-ethylthiourea, N-propylthiourea, N-butylthiourea, N-laurylthiourea, N-phenylthiourea, N-cyclohexylthiourea, N,N-dimethylthiourea, N,N-diethylthiourea, N,N-dipropylthiourea, N,N-dibutylthiourea, N,N-dilaurylthiourea, N,N-diphenylthiourea, N,N-dicyclohexylthiourea, trimethylthiourea, tetramethylthiourea, N-acetylthiourea, N-benzoylthiourea, 1-allyl-3-(2-hydroxyethyl)-2-thiourea, 1-(2-tetrahydrofurfuryl)-2-thiourea, N-tert-butyl-N'-isopropylthiourea, 2-pyridylthiourea, and the like.

The thiourea derivative may be used alone, or in combination of two or more. Among these, N-benzoylthiourea is preferable in terms of improving the curability of the dental adhesive composition.

The content of the thiourea derivative in the first agent is not particularly limited, but is preferably 0.1 mass% or more and 5 mass% or less, more preferably 0.1 mass% or more and 3 mass% or less, still more preferably 0.1 mass% or more and 1 mass% or less, based on 100 mass% of the first agent.

When the first agent contains a plurality of types of thiourea derivatives, it is preferable that the total content satisfies the above range.

When the content of the thiourea derivative in the first agent is 0.1 mass% or more, the curability of the dental adhesive composition is further improved, and when the content is 5 mass% or less, the solubility of the thiourea derivative in the (meth)acrylate in the dental adhesive composition is improved.

### (D-2) Vanadium Compound

Among the chemical polymerization initiators, the vanadium compound functions as a reducing agent.

Examples of the vanadium compound include, but are not particularly limited to, oxovanadium oxalate, vanadyl acetylacetonate, vanadium acetylacetonate, vanadyl stearate, vanadium naphthenate, vanadium benzoylacetonate, and the like. The vanadium compound may be used in combination of two or more. Among these, vanadyl acetylacetonate is preferable in terms of the curability of the dental adhesive composition.

The content of the vanadium compound in the first agent is not particularly limited, but is preferably 0.001 mass% or more and 5 mass% or less, more preferably 0.0015 mass% or more and 1 mass% or less, and still more preferably 0.002 mass% or more and 0.1 mass% or less, based on 100 mass% of the first agent.

When the first agent contains a plurality of types of vanadium compounds, it is preferable that the total content satisfies the above range.

When the content of the vanadium compound in the first agent is 0.001 mass% or more, the curability of the dental adhesive composition is further improved, and when the content is 5 mass% or less, the storage stability of the dental adhesive composition is further improved.

### (D-3) Organic Peroxide

Among the chemical polymerization initiators, the organic peroxide functions as an oxidizing agent.

Examples of the organic peroxide include benzoyl peroxide, cumene hydroperoxide, t-butyl hydroperoxide, t-amyl hydroperoxide, 1,1,3,3-tetramethylbutyl hydroperoxide, 2,5-dimethyl-2,5-di(hydroperoxide) hexane, p-diisopropylbenzene monohydroperoxide, p-methane hydroperoxide, pinane hydroperoxide, and the like.

The organic peroxide may be used alone, or in combination of two or more. Among these, cumene hydroperoxide is preferable in terms of the curability of the dental adhesive composition.

The content of the organic peroxide in the first agent is not particularly limited, but is preferably 0.01 mass% or more and 10 mass% or less, more preferably 0.05 mass% or more and 5 mass% or less, and still more preferably 0.1 mass% or more and 3 mass% or less, based on 100 mass% of the first agent.

When the first agent contains a plurality of types of organic peroxides, it is preferable that the total content satisfies the above range.

When the content of the organic peroxide in the first agent is 0.01 mass% or more, the curability of the dental adhesive composition is further improved, and when the content is 10 mass% or less, the working time with the dental adhesive composition is increased, thereby ensuring sufficient working time.

### (E) Photopolymerization Initiator

Examples of the photopolymerization initiator include, but are not particularly limited to, camphorquinone (CQ), a tertiary amine, 2,4,6-trimethylbenzoyldiphenylphosphine oxide (TPO), phenylbis(2,4,6-trimethylbenzoyl) phosphine oxide, benzyl ketal, diacetyl ketal, benzyl dimethyl ketal, benzyl diethyl ketal, benzyl bis(2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl) ketone, 4,4'-bis(diethylamino) benzophenone, and the like.

A tertiary amine, one type of photopolymerization initiator, will be described.

### (E-1) Tertiary Amine

Among the photopolymerization initiators, the tertiary amine functions as a reducing agent.

Examples of the tertiary amine include, but are not particularly limited to, a tertiary aliphatic amine and a tertiary aromatic amine.

Examples of the tertiary aliphatic amine include N,N-dimethylaminoethyl methacrylate, triethanolamine, and the like.

Examples of the tertiary aromatic amine include alkyl 4-dialkylaminobenzoate, 7-dimethylamino-4-methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

Among these, the tertiary amine is preferably the tertiary aromatic amine, and more preferably an alkyl 4-dialkylaminobenzoate.

Examples of the alkyl 4-dialkylaminobenzoate include methyl 4-dimethylaminobenzoate, ethyl 4-dimethylaminobenzoate (EPA), propyl 4-dimethylaminobenzoate, amyl 4-dimethylaminobenzoate, isoamyl 4-dimethylaminobenzoate, ethyl 4-diethylaminobenzoate, propyl 4-diethylaminobenzoate, and the like.

The tertiary amine may be used alone, or in combination of two or more.

The photopolymerization initiator may be used alone, or in combination of two or more. Among these, camphorquinone (CQ), ethyl 4-dimethylaminobenzoate (EPA), and 2,4,6-trimethylbenzoyldiphenylphosphine oxide (TPO) are preferable in terms of improving the curability of the dental adhesive composition.

The content of the photopolymerization initiator in the first agent is not particularly limited, but is, for example, preferably 0.01 mass% or more and 10 mass% or less, more preferably 0.05 mass% or more and 8 mass% or less, and still more preferably 1 mass% or more and 5 mass% or less, based on 100 mass% of the first agent.

When the first agent contains a plurality of types of photopolymerization initiators, it is preferable that the total content satisfies the above range.

When the content of the photopolymerization initiator in the first agent is 0.01 mass% or more, the curability of the dental adhesive composition is further improved, and when the content is 10 mass% or less, the storage stability of the dental adhesive composition is further improved.

The polymerization initiator as described above may be contained in either the first agent or the second agent, or in both of the agents. Thus, it is preferable that at least one of the first agent or the second agent contains the polymerization initiator. Because the dental adhesive composition according to the present embodiment is in many cases cured by irradiation with ultraviolet light or the like, the photopolymerization initiator described above may be particularly preferably used as the polymerization initiator. Therefore, it is preferable that at least one of the first agent or the second agent contains the photopolymerization initiator.

### (F) Polymerization Inhibitor

Examples of the polymerization inhibitor include dibutylhydroxytoluene (2,6-di-tert-butyl-p-cresol) (BHT), 6-tert-butyl-2,4-xylenol, and the like.

The polymerization inhibitor may be used alone, or in combination of two or more. Among these, dibutylhydroxytoluene (BHT) is preferable in terms of improving the curability of the dental adhesive composition.

The content of the polymerization inhibitor in the first agent is not particularly limited, but is, for example, preferably 0.01 mass% or more and 5 mass% or less, more preferably 0.05 mass% or more and 3 mass% or less, still more preferably 0.1 mass% or more and 1 mass% or less, based on 100 mass% of the first agent.

When the first agent contains a plurality of types of polymerization inhibitors, it is preferable that the total content satisfies the above range.

When the content of the photopolymerization initiator in the first agent is 0.01 mass% or more and 5 mass% or less, the storage stability of the dental adhesive composition is improved.

### (G) Filler

Examples of the filler include, but are not particularly limited to, colloidal silica; fine particle silica in which the surface is hydrophobized (hydrophobic fumed silica); aluminum oxide; fluoroaluminosilicate glass; barium glass; and the like. Among these, hydrophobic fumed silica (for example, Aerosil (registered trademark)) is preferable. The filler may be used alone, or in combination of two or more.

The content of the filler in the first agent is preferably, for example, 0.1 mass% or more and 30 mass% or less, more preferably 0.5 mass% or more and 20 mass% or less, and still more preferably 1 mass% or more and 10 mass% or less, based on 100 mass% of the first agent.

When the first agent contains a plurality of types of fillers, it is preferable that the total content satisfies the above range.

When the content of the filler in the first agent is 0.1 mass% or more, the viscosity of the dental adhesive composition increases, and the operability of the dental adhesive composition can be improved. When the content of the filler in the first agent is 30 mass% or less, the viscosity of the dental adhesive composition does not become too high, and the high operability of the dental adhesive composition can be maintained.

### (H) Solvent

Examples of the solvent include, but are not particularly limited to, one or more solvents selected from water and an organic solvent. The first agent may also contain both water and the organic solvent as the solvent.

### (H-1) Organic Solvent

Examples of the organic solvent include ethanol, acetone, 2-propanol, ethyl methyl ketone, and the like.

The content of the organic solvent in the first agent is not particularly limited, but is preferably 10 mass% or more and 50 mass% or less, and more preferably 20 mass% or more and 40 mass% or less, based on 100 mass% of the first agent.

When the first agent contains a plurality of types of organic solvents, it is preferable that the total content satisfies the above range.

When the content of the organic solvent in the first agent is 10 mass% or more, the uniformity of the dental adhesive composition is improved, and when the content is 50 mass% or less, the adhesiveness of the dental adhesive composition as a primer is improved.

### (H-2) Water

The water is not particularly limited, but is preferably ion-exchanged water or distilled water. When the first agent contains water, the acid group of the (meth)acrylate having the acid group is dissociated. Therefore, the solubility of the smear layer on the tooth surface; and the tooth demineralization and the permeability to the tooth of the dental adhesive composition as a primer, are improved. Therefore, when the tooth is pretreated with the first agent, the permeability to the tooth of the dental adhesive composition as a primer is improved, and the adhesiveness of the dental adhesive composition is improved.

Therefore, it is preferable that the first agent further contains water.

The content of water in the first agent is not particularly limited, but is, for example, preferably 1 mass% or more and 50 mass% or less, and more preferably 5 mass% or more and 40 mass% or less, based on 100 mass% of the first agent. When the content of water in the first agent is 1 mass% or more and 50 mass% or less, the solubility of the smear layer on the tooth; and the tooth demineralization and the permeability to the tooth of the dental adhesive composition as a primer, are improved.

### (1-2) pH

The pH of the first agent is preferably 1.2 or more and 4.0 or less, and more preferably 1.5 or more and 3.0 or less. By setting the pH of the first agent to 1.2 or more and 4.0 or less, the solubility of the smear layer on the tooth and the tooth demineralization are improved, and the adhesiveness of the dental adhesive composition is improved.

### (2) Second Agent

In the dental adhesive composition according to the present embodiment, the second agent is also referred to as a bonding agent or bond, and contains a (meth)acrylate.

### (2-1) Component Contained in Second Agent

Hereinafter, the components contained in the second agent will be described.

### (A) (Meth)acrylate

The (meth)acrylate in the second agent is not particularly limited, but is preferably a di(meth)acrylate, more preferably a di(meth)acrylate having a molecular weight of 400 or more, and a di(meth)acrylate having a molecular weight of less than 400. As the (meth)acrylate, a polyfunctional (meth)acrylate may be preferably used.

Examples of the di(meth)acrylate having a molecular weight of 400 or more include an aliphatic di(meth)acrylate having a molecular weight of 400 or more, an aromatic di(meth)acrylate having a molecular weight of 400 or more, and the like.

Specific examples of the aliphatic di(meth)acrylate having a molecular weight of 400 or more include 1,6-bis-[2-methacryloyloxyethoxycarbonylamino]-2,4,4-trimethylhexane (UDMA), polyethylene glycol #400 dimethacrylate, polyethylene glycol #600 dimethacrylate, polyethylene glycol #1000 dimethacrylate, and the like.

Specific examples of the aromatic di(meth)acrylate having a molecular weight of 400 or more include bisphenol A diglycidyl methacrylate (Bis-GMA), 2-(4-(meth)acryloyloxydiethoxyphenyl)-2-(4-(meth)acryloyloxytriethoxyphenyl) propane, 2-(4-(meth)acryloyloxydipropoxyphenyl)-2-(4-(meth)acryloyloxytriethoxyphenyl) propane, 2,2-bis(4-(meth)acryloyloxypropoxyphenyl) propane, 2,2-bis(4-(meth)acryloyloxyisopropoxyphenyl) propane, and the like.

The di(meth)acrylate having a molecular weight of less than 400 is not particularly limited, but is preferably a di(meth)acrylate having a molecular weight of less than 300.

Examples of the di(meth)acrylate having a molecular weight of less than 300 include 2-hydroxy-1,3-dimethacryloyloxypropane, diethylene glycol dimethacrylate, triethylene glycol dimethacrylate, polyethylene glycol #200 dimethacrylate, 1,6-hexanediol dimethacrylate, 1,9-nonanediol dimethacrylate, 1,10-decanediol dimethacrylate, neopentyl glycol dimethacrylate, and the like. Among these, a di(meth)acrylate containing a hydroxyl group is preferable, and 2-hydroxy-1,3-dimethacryloyloxypropane is more preferable.

The content of the (meth)acrylate in the second agent is not particularly limited, but is, for example, preferably 50 mass% or more and 99 mass% or less, and more preferably 60 mass% or more and 95 mass% or less, based on 100 mass% of the second agent.

When the second agent contains a plurality of types of (meth)acrylate, it is preferable that the total content satisfies the above range.

When the content of the (meth)acrylate in the second agent is 50 mass% or more and 99 mass% or less, the adhesiveness of the dental adhesive composition is improved, and the mechanical strength of the cured body of the dental adhesive composition is improved.

The second agent may contain both the di(meth) acrylate having a molecular weight of 400 or more and the di(meth)acrylate having a molecular weight of less than 400.

A ratio (B/A) of the content (B) of the di(meth)acrylate having a molecular weight of less than 400 to the content (A) of the di(meth)acrylate having a molecular weight of 400 or more in the second agent is not particularly limited, but is preferably 0.5 or more and 2 or less, and more preferably 0.8 or more and 1.4 or less by mass. By setting the ratio of the content of the di(meth)acrylate having a molecular weight of less than 400 to the content of the di(meth)acrylate having a molecular weight of 400 or more in the second agent to 0.5 or more and 2 or less, the adhesiveness of the dental adhesive composition is improved.

The second agent may contain other components as long as they do not detract from the purpose of the present invention. Examples of the other components contained in the second agent include a photopolymerization initiator, a filler, a polymerization inhibitor, and the like.

Hereinafter, the above components will also be described.

### (B) Photopolymerization Initiator

When the second agent contains the photopolymerization initiator, the photopolymerization initiator is not particularly limited, but is, for example, α-diketone compounds such as camphorquinone, a tertiary amine, acylphosphine oxide compounds such as 2,4,6-trimethylbenzoyldiphenylphosphine oxide, 2,4,6-trimethoxybenzoyldiphenylphosphine oxide, 2,6-dimethylbenzoyldiphenylphosphine oxide, 2,6-dimethoxybenzoyldiphenylphosphine oxide, and the like, benzyl ketal, diacetyl ketal, benzyl dimethyl ketal, benzyl diethyl ketal, benzyl bis(2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl) ketone, 4,4'-bis(diethylamino) benzophenone, compounds containing an azide group, and the like. The photopolymerization initiator may be used alone, or in combination of two or more.

When the tertiary amine is used as the photopolymerization initiator in the second agent, the tertiary amine is not particularly limited, but for example, an aromatic tertiary amine such as ethyl 4-dimethylaminobenzoate and the like may be suitably used.

The content of the photopolymerization initiator in the second agent is not particularly limited, but is, for example, preferably 0.01 mass% or more and 8 mass% or less, and more preferably 3 mass% or more and 6 mass% or less, based on 100 mass% of the second agent.

When the second agent contains a plurality of types of photopolymerization initiators, it is preferable that the total content satisfies the above range.

When the content of the photopolymerization initiator in the second agent is 0.01 mass% or more and 8 mass% or less, the adhesiveness of the dental adhesive composition is improved, and the storage stability of the dental adhesive composition as a bonding agent is improved.

### (C) Filler

The second agent may contain the filler. The content of the filler in the second agent is preferably 0 mass% or more and 50 mass% or less, more preferably 0.1 mass% or more and 20 mass% or less. That is, when the second agent contains the filler, the content of the filler is preferably not more than 50 mass%.

When the second agent contains a plurality of types of fillers, it is preferable that the total content satisfies the above range.

When the content of the filler in the second agent is 50 mass% or less, the dispersibility of the second agent is improved, and the storage stability of the dental adhesive composition as a bonding agent is improved. When the content of the filler in the second agent is 50 mass% or less, the proportion of the polymerization component is also increased, and therefore, the adhesiveness is particularly improved.

As the filler, the same filler as described for the first agent may be suitably used, and the description thereof will be omitted here.

### (D) Polymerization Inhibitor

Examples of the polymerization inhibitor used for the second agent include, but are not particularly limited to, dibutylhydroxytoluene (2,6-di-tert-butyl-p-cresol) (BHT), 2,6-t-butyl-2,4-xylenol, and the like. When the second agent contains the polymerization inhibitor, the content of the polymerization inhibitor in the second agent is not particularly limited, but is, for example, preferably 0.01 mass% or more and 3 mass% or less, and more preferably 0.1 mass% or more and 1 mass% or less, based on 100 mass% of the second agent.

When the second agent contains a plurality of types of polymerization inhibitors, it is preferable that the total content satisfies the above range.

### (3) Application of Dental Adhesive Composition

The dental adhesive composition according to the present embodiment may be used by applying the first agent to a target, followed by air blowing, and then applying the second agent to the target.

That is, the method of using the dental adhesive composition according to the present embodiment may include a first agent application step of applying the first agent to the target to be adhered, an air blow step of air blowing on the applied surface of the first agent, and a second agent application step of applying the second agent on the applied surface of the first agent.

After the second application step, an arrangement and curing step may be further performed in which an object to be adhered to the target is arranged on the applied surface of the second agent, and the second agent is cured. Because the target to be adhered and the object to be adhered to the target can be adhered and an adhesive structure can be manufactured, the method including the above step may also be referred to as a method of manufacturing an adhesive structure.

The air blowing may be performed by a publicly-known method, and may be performed immediately after the application of the first agent.

The method of applying the first agent to the target may be freely determined, and for example, the first agent may be applied to the target by a publicly-known method. The method of applying the second agent to the target may also be freely determined, and for example, the second agent may be applied to the target to which the first agent has been applied, by a publicly-known method.

When the dental adhesive composition according to the present embodiment is used in such a manner, the dental adhesive composition according to the present embodiment can be used in the same manner as a conventional dental adhesive composition provided as a two-part formulation, and therefore can be easily handled.

The dental adhesive composition according to the present embodiment can exhibit high adhesiveness to any of ceramics, enamel, and dentin. Therefore, according to the dental adhesive composition according to the present embodiment, for example, the adhesiveness between dental prosthetics made of ceramic materials and a tooth can be increased.

The application of the dental adhesive composition according to the present embodiment is not particularly limited, but it can be used for various dental materials, for example.

Examples of the application of the dental adhesive composition according to the present embodiment include dental cement, dental adhesive, dental temporary sealing material, dental coat material, dental hard resin, dental cutting resin material, dental temporary restorative material, dental filler, dentifrice, and the like. Among these, the dental adhesive composition according to the present embodiment is suitably used for a dental adhesive.

### Examples

Hereinafter, the present invention will be further described with reference to Examples and Comparative Examples. In the following, "parts" or "%" are based on mass unless otherwise specified. Various tests and evaluations are performed according to the following methods.

### (1) Evaluation Method

The evaluation method of the dental adhesive composition prepared in the following Examples and Comparative Examples will be described.

### (1-1) Adhesive Strength to Ceramic

Test specimens were prepared and evaluated in the following order (A) to (I).
(A) A ceramic was polished with #600 water-resistant abrasive paper.
(B) The polished surface of the ceramic was sandblasted with a sandblaster. At this time, the pressure of sandblasting was set to 0.2 MPa.
(C) The ceramic was immersed in water, and ultrasonic cleaning for 10 minutes was performed twice.
(D) A first agent was applied to the sandblasted surface, and the surface was immediately dried by air blowing.
(E) After the application of the first agent, a second agent was applied on the dried surface with an applicator, and the adhesive surface was flattened immediately by air blowing.
(F) A mold with a circular hole of 2.38 mm in diameter manufactured by Ultradent Products, Inc. was placed on the applied surface of the first agent and the second agent, and the mold was irradiated with light using a light irradiator (G-Light Prima II Plus, manufactured by GC Corporation) for 10 seconds.
(G) The hole of the mold was filled with a composite resin (Clearfil AP-X, manufactured by Kuraray Noritake Dental Inc.) and further irradiated with light for 20 seconds.
(H) By immersing in water at 37°C for 24 hours, test specimens were obtained.
(I) Five prepared test specimens were subjected to shear tests using an autograph (EZ-S, manufactured by Shimadzu Corporation) at a crosshead speed of 1 mm/min. The average value of the shear bond strength of the five test specimens was obtained to evaluate the adhesiveness of the composite resin to the ceramic when the dental adhesive composition was used.

The first agent respectively prepared in the Examples and Comparative Examples of the formulations presented in Tables 1 and 2 was used. The second agent used in the Examples and Comparative Examples was any of those indicated by A to C in the columns of "second agent" in Tables 1 and 2. The specific formulations of the second agent are presented in Table 3.

Five test specimens prepared in the Examples and Comparative Examples under the same conditions were evaluated as described above.

The evaluation criteria for the adhesiveness of the composite resin to the ceramic when the dental adhesive composition is used are as follows. When the evaluation is excellent or good, it is determined that the adhesive strength to the ceramic is sufficient. When the evaluation is poor, it is determined that the adhesive strength to the ceramic is insufficient. The average values for the five test specimens are presented in the columns of "measured value" of "adhesive strength to ceramics" in Tables 1 and 2 and the evaluation results are presented in the columns of "evaluation".

### [Evaluation Criteria]

Excellent: the average of the shear bond strength is 20 MPa or more
Good: the average of the shear bond strength is 17.5 MPa or more and less than 20 MPa
Poor: the average of the shear bond strength is less than 17.5 MPa

### (1-2) Adhesive Strength to Enamel and Adhesive Strength to Dentin

Test specimens were prepared and evaluated in the following order (A) to (G).
(A) The enamel or dentin of a bovine tooth was polished with #400 water-resistant abrasive paper, and the fresh surface was exposed. When the enamel of the bovine tooth was used, the adhesive strength to enamel was evaluated. When the dentin was used, the adhesive strength to dentin was evaluated.
(B) A first agent was applied to the exposed fresh surface, and the surface was immediately dried by air blowing.
(C) After the application of the first agent, a second agent was applied on the dried surface with an applicator, and the adhesive surface was flattened immediately by air blowing.
(D) A mold with a circular hole of 2.38 mm in diameter manufactured by Ultradent Products, Inc. was placed on the applied surface of the first agent and the second agent, and the mold was irradiated with light using a light irradiator (G-Light Prima II Plus, manufactured by GC Corporation) for 10 seconds.
(E) The hole of the mold was filled with a composite resin (Clearfil AP-X, manufactured by Kuraray Noritake Dental Inc.) and further irradiated with light for 20 seconds.
(F) By immersing in water at 37°C for 24 hours, test specimens were obtained.
(G) Five prepared test specimens were subjected to shear tests using an autograph (EZ-S, manufactured by Shimadzu Corporation) at a crosshead speed of 1 mm/min. The average value of the shear bond strength of the five test specimens was obtained to evaluate the adhesiveness of the composite resin to the enamel and dentin when the dental adhesive composition was used.

The first agent respectively prepared in the Examples and Comparative Examples of the formulations presented in Tables 1 and 2 was used. The second agent used in the Examples and Comparative Examples was any of those indicated by A to C in the columns of "second agent" in Tables 1 and 2. The specific formulations of the second agent are presented in Table 3.

Five test specimens prepared in the Examples and Comparative Examples under the same conditions were evaluated as described above. In each of the Examples and Comparative Examples, five test specimens for enamel and five test specimens for dentin were prepared.

The evaluation criteria for the adhesiveness of the composite resin to the tooth (enamel and dentin) when the dental adhesive composition is used are as follows. When the evaluation is excellent or good, it is determined that the adhesive strength to each of the tooth is sufficient. When the evaluation is poor, it is determined that the adhesive strength to each of the tooth is insufficient. The average values for the five test specimens are respectively presented in the columns of "measured value" of "adhesive strength to enamel" and "adhesive strength to dentin" in Tables 1 and 2 and the evaluation results are presented in the columns of "evaluation".

### [Evaluation Criteria] (Adhesive Strength to Enamel)

Excellent: the average of the shear bond strength is 35 MPa or more
Good: the average of the shear bond strength is 25 MPa or more and less than 35 MPa
Poor: the average of the shear bond strength is less than 25 MPa

### [Evaluation Criteria] (Adhesive Strength to Dentin)

Excellent: the average of the shear bond strength is 40 MPa or more
Good: the average of the shear bond strength is 35 MPa or more and less than 40 MPa
Poor: the average of the shear bond strength is less than 35 MPa

### (2) Preparation Condition of Dental Adhesive Composition

### [Example 1]

### (A) Sample (First Agent)

The first agent constituting the dental adhesive composition provided as a two-part formulation was prepared to have the formulation presented in Table 1. The pH of the first agent formulated in Table 1 was 1.5. The pH value of the first agent was the same in the following other Examples and Comparative Examples.

The specifications of the components indicated by abbreviations or product names in Table 1 are as follows.
GDMA: 2-hydroxy-1,3-dimethacryloyloxypropane
TEGDMA: triethylene glycol dimethacrylate
4-META: 4-methacryloyloxyethyl trimellitic anhydride
MDP: 10-methacryloyloxydecyl dihydrogen phosphate
CQ: camphorquinone
EPA: ethyl 4-dimethylaminobenzoate
TPO: 2,4,6-trimethylbenzoyldiphenylphosphine oxide
BHT: dibutylhydroxytoluene
AEROSIL (registered trademark) R972: fine particle silica surface-treated with dimethyldichlorosilane (DDS) (manufactured by Nippon Aerosil Co., Ltd.)

Among the silicone compounds, KP-410, KP-422, KP-423, and DMS-U21 are linear siloxanes satisfying the aforementioned general formula (1). Among the silicone compounds, KL-700 is a siliconecontaining acrylic polymer that does not satisfy the aforementioned general formula (1).

### (B) Second Agent

The second agent constituting the dental adhesive composition provided as a two-part formulation was prepared to have the formulation indicated by A in Table 3.

The specifications of the components indicated by abbreviations or product names in Table 3 are as follows.
GDMA: 2-hydroxy-1,3-dimethacryloyloxypropane
UDMA: 1,6-bis-[2-methacryloyloxyethoxycarbonylamino]-2,4,4-trimethylhexane
TEGDMA: triethylene glycol dimethacrylate
Bis-GMA: bisphenol A diglycidyl methacrylate
AEROSIL (registered trademark) RX50: fine particle silica surface-treated with hexamethyldisilazane (manufactured by Nippon Aerosil Co., Ltd.)
AEROSIL (registered trademark) RX200: fine particle silica surface-treated with hexamethyldisilazane (manufactured by Nippon Aerosil Co., Ltd.)
CQ: camphorquinone
EPA: ethyl 4-dimethylaminobenzoate
TPO: 2,4,6-trimethylbenzoyldiphenylphosphine oxide
BHT: dibutylhydroxytoluene

Using the first agent and the second agent obtained, the above evaluations were performed. The evaluation results are presented in Table 1.

### [Example 2 to Example 15]

Dental adhesive compositions were prepared by the same procedure as in Example 1, except that the formulation of the first agent was the formulation presented in Tables 1 and 2, and any of A to C presented in the columns of "second agent" in Tables 1 and 2 was used as the second agent, and the evaluations described above were performed. The specific formulations of the second agent are presented in Table 3. The evaluation results are presented in Tables 1 and 2.

### [Comparative Example 1 to Comparative Example 3]

Dental adhesive compositions were prepared by the same procedure as in Example 1, except that the formulation of the first agent was the formulation presented in Table 2, and A or B presented in the columns of "second agent" in Table 2 was used as the second agent, and the evaluations described above were performed. The specific formulations of the second agent are presented in Table 3. The evaluation results are presented in Table 2.

**[Table 1]**

| | | | EXAMPLE 1 | EXAMPLE 2 | EXAMPLE 3 | EXAMPLE 4 | EXAMPLE 5 | EXAMPLE 6 | EXAMPLE 7 | EXAMPLE 8 |
|---|---|---|---|---|---|---|---|---|---|---|
| FIRST AGENT FORMULATION (MASS %) | ORGANIC SOLVENT | ACETONE | 30 | 31 | 28 | 35 | 30 | 31 | 28 | 25 |
| | WATER | DISTILLED WATER | 20 | 23 | 24 | 14 | 23 | 23 | 19 | 15 |
| | (METH)ACRYLATE HAVING NO ACID GROUP | GDMA | 15 | 11 | 12 | 10 | 13 | 13 | 15 | 16 |
| | | TEGDMA | 5 | 5 | 5 | 5 | 5 | 5 | 5 | 5 |
| | (METH)ACRYLATE HAVING ACID GROUP | 4-META | 8 | 8 | 7 | 7 | 12 | 10 | 9 | 10 |
| | | MDP | 10 | 7 | 8 | 8 | 5 | 5 | 7 | 7 |
| | POLYMERIZATION INITIATOR | CQ | 2 | 1.6 | 1 | 1 | 2.6 | 2 | 1.7 | 1 |
| | | EPA | 1 | 1 | 1 | 1 | 1 | 1 | 1 | 1 |
| | | TPO | 2 | 2 | 2 | 2 | 2 | 2 | 2 | 2 |
| | POLYMERIZATION INHIBITOR | BHT | 1 | 1 | 1 | 1 | 1 | 1 | 1 | 1 |
| | FILLER | AEROSIL R972 | 5 | 5 | 5 | 5 | 5 | 5 | 5 | 5 |
| | SILICONE COMPOUND | KP-410 | 1.2 | 4.4 | 6.5 | 11.2 | - | - | - | - |
| | | KP-422 | - | - | - | - | 0.4 | 2.2 | 7.8 | 12.3 |
| | | KP-423 | - | - | - | - | - | - | - | - |
| | | DMS-U21 | - | - | - | - | - | - | - | - |
| | | KL-700 | - | - | - | - | - | - | - | - |
| | TOTAL | | 100 | 100 | 100 | 100 | 100 | 100 | 100 | 100 |
| SECOND AGENT | | | A | B | A | A | B | A | A | A |
| EVALUATION RESULT | ADHESIVE STRENGTH TO CERAMICS [MPa] | MEASURED VALUE [MPa] | 20.0 | 21.2 | 21.7 | 17.7 | 19.9 | 20.5 | 21.6 | 21.0 |
| | | EVALUATION | EXCELLENT | EXCELLENT | EXCELLENT | GOOD | GOOD | EXCELLENT | EXCELLENT | EXCELLENT |
| | ADHESIVE STRENGTH TO ENAMEL [MPa] | MEASURED VALUE [MPa] | 37.2 | 35.5 | 33.4 | 40.0 | 34.1 | 35.6 | 34.8 | 32.6 |
| | | EVALUATION | EXCELLENT | EXCELLENT | GOOD | EXCELLENT | GOOD | EXCELLENT | GOOD | GOOD |
| | ADHESIVE STRENGTH TO DENTIN [MPa] | MEASURED VALUE [MPa] | 42.9 | 43.6 | 41.1 | 43.3 | 42.8 | 40.1 | 42.1 | 43.9 |
| | | EVALUATION | EXCELLENT | EXCELLENT | EXCELLENT | EXCELLENT | EXCELLENT | EXCELLENT | EXCELLENT | EXCELLENT |

**[Table 2]**

| | | | EXAMPLE 9 | EXAMPLE 10 | EXAMPLE 11 | EXAMPLE 12 | EXAMPLE 13 | EXAMPLE 14 | EXAMPLE 15 | COMPARATIVE EXAMPLE 1 | COMPARATIVE EXAMPLE 2 | COMPARATIVE EXAMPLE 3 |
|---|---|---|---|---|---|---|---|---|---|---|---|---|
| **FIRST AGENT** FORMULATION (MASS %) | ORGANIC SOLVENT | ACETONE | 27 | 38 | 34 | 23 | 36 | 27 | 37 | 30 | 27 | 30 |
| | WATER | DISTILLED WATER | 20 | 18 | 19 | 17 | 15 | 19 | 15 | 20 | 21 | 22 |
| | (METH)ACRYLATE HAVING NO ACID GROUP | GDMA | 15 | 13 | 13 | 15 | 14 | 15 | 9 | 11 | 11 | 15 |
| | | TEGDMA | 5 | 5 | 5 | 5 | 5 | 5 | 5 | 5 | 5 | 5 |
| | (METH)ACRYLATE HAVING ACID GROUP | 4-META | 11 | 7 | 9 | 10 | 9 | 9 | 8 | 11 | 11 | 8 |
| | | MDP | 10 | 5 | 5 | 10 | 11 | 10 | 6 | 8 | 8 | 9 |
| | POLYMERIZATION INITIATOR | CQ | 2 | 1.9 | 1.9 | 1 | 1 | 0.6 | 1 | 1.6 | 1.5 | 2 |
| | | EPA | 1 | 1 | 1 | 1 | 1 | 1 | 1 | 1 | 1 | 1 |
| | | TPO | 2 | 2 | 2 | 2 | 2 | 2 | 2 | 2 | 2 | 2 |
| | POLYMERIZATION INHIBITOR | BHT | 1 | 1 | 1 | 1 | 1 | 1 | 1 | 1 | 1 | 1 |
| | FILLER | AEROSIL R972 | 5 | 5 | 5 | 5 | 5 | 5 | 5 | 5 | 5 | 5 |
| | SILICONE COMPOUND | KP-410 | - | - | - | - | - | - | - | - | - | - |
| | | KP-422 | - | - | - | - | - | - | - | - | - | - |
| | | KP-423 | 10 | 3.1 | 4.6 | 9.7 | - | - | - | - | - | - |
| | | DMS-U21 | - | - | - | - | 0.5 | 5.4 | 10.1 | - | - | - |
| | | KL-700 | - | - | - | - | - | - | - | 4.4 | 6.5 | - |
| | TOTAL | | 100 | 100 | 100 | 100 | 100 | 100 | 100 | 100 | 100 | 100 |
| SECOND AGENT | | | A | C | A | A | B | B | C | A | B | A |
| EVALUATION RESULT | ADHESIVE STRENGTH TO CERAMICS [MPa] | MEASURED VALUE [MPa] | 22.2 | 20.3 | 18.9 | 18.1 | 20.5 | 21.0 | 19.8 | 14.1 | 12.6 | 17.4 |
| | | EVALUATION | EXCELLENT | EXCELLENT | GOOD | GOOD | EXCELLENT | EXCELLENT | GOOD | POOR | POOR | POOR |
| | ADHESIVE STRENGTH TO ENAMEL [MPa] | MEASURED VALUE [MPa] | 33.1 | 32.9 | 35.0 | 35.7 | 34.7 | 32.5 | 35.4 | 28.2 | 25.0 | 35.8 |
| | | EVALUATION | GOOD | GOOD | EXCELLENT | EXCELLENT | GOOD | GOOD | EXCELLENT | GOOD | GOOD | EXCELLENT |
| | ADHESIVE STRENGTH TO DENTIN [MPa] | MEASURED VALUE [MPa] | 42.2 | 43.6 | 40.2 | 41.3 | 45.0 | 42.3 | 40.7 | 22.6 | 21.4 | 40.7 |
| | | EVALUATION | EXCELLENT | EXCELLENT | EXCELLENT | EXCELLENT | EXCELLENT | EXCELLENT | EXCELLENT | POOR | POOR | EXCELLENT |

**[Table 3]**

| | | | A | B | C |
|---|---|---|---|---|---|
| FORMULATION (MASS %) | (METH)ACRYLATE | GDMA | 45 | - | 45 |
| | | UDMA | 30 | 30 | 30 |
| | | TEGDMA | - | 45 | - |
| | | Bis-GMA | 13 | 13 | 13 |
| | FILLER | AEROSIL RX50 | 5 | 5 | - |
| | | AEROSIL RX200 | - | - | 5 |
| | POLYMERIZATION INITIATOR | CQ | 2 | 2 | 2 |
| | | EPA | 2 | 2 | 2 |
| | | TPO | 2 | 2 | 2 |
| | POLYMERIZATION INHIBITOR | BHT | 1 | 1 | 1 |
| | TOTAL | | 100 | 100 | 100 |

From the results presented in Tables 1 and 2, it can be seen that when the dental adhesive compositions of Examples 1 to 15 are used, all items are excellent or good, and the dental adhesive compositions have good adhesiveness to ceramics.

In contrast, it was observed that the dental adhesive compositions of Comparative Examples 1 to 3, which do not contain the linear siloxane represented by the aforementioned general formula (1) in the first agent, do not have sufficient adhesive strength to ceramics.

While embodiments of the invention have been described, the invention is not limited to specific embodiments, and various modifications and variations are possible within the scope of the invention as claimed.

The present application is based on and claims priority to Japanese Patent Application No. 2022-060618, filed March 31, 2022, the contents of which are incorporated herein by reference in their entirety.

## Claims

1. A dental adhesive composition provided as a two-part formulation including a first agent and a second agent, wherein
the first agent contains a (meth)acrylate having an acid group and a linear siloxane represented by a general formula (1) 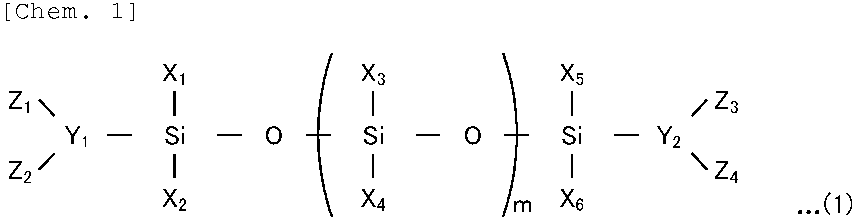
(in the general formula (1), X₁ to X₆ are independently an alkyl group or an ethylenically unsaturated group having 1 or more and 6 or less carbon atoms; m is an integer of 0 or more and 500 or less; in a case where m is 2 or more, X₃ and X₄ are same or different for each repeating unit; Y₁ and Y₂ are independently a linear hydrocarbon group or a branched hydrocarbon group having 1 or more and 20 or less carbon atoms; Z₁ to Z₄ are independently a hydrogen atom or an ethylenically unsaturated group; and at least one of X₁ to X₆ or Z₁ to Z₄ is an ethylenically unsaturated group), and
the second agent contains a (meth)acrylate.

2. The dental adhesive composition according to claim 1, wherein
the first agent further contains water, and
at least one of the first agent or the second agent contains a photopolymerization initiator.

3. The dental adhesive composition according to claim 1 or 2, wherein the dental adhesive composition is used by applying the first agent to a target, followed by air blowing, and then applying the second agent to the target.
